# FASCICULE DE BREVET EUROPEEN

(11) **EP 3 407 980 B1**
(45) Date de publication et mention de la délivrance du brevet: **17.04.2024**
(21) Numéro de dépôt: 17701543.5
(22) Date de dépôt: 27.01.2017
(51) Int. Cl.: A61Q 17/00, A61Q 19/08, A61K 8/73, A61K 36/48, A61K 36/482, A61K 36/489, A61K 8/9717, A61K 8/9789, A61K 36/04

(54) **AGENT COSMETIQUE TENSEUR ET/OU FILMOGENE CONSTITUE PAR DES GALACTOMANNANES ET DES GALACTANES SULFATES RETICULES**
STRAFFENDES UND/ODER FILMBILDENDES KOSMETISCHES MITTEL AUS GALACTOMANNANEN UND VERNETZTEN SULFATIERTEN GALACTANEN
TIGHTENING AND/OR FILM-FORMING COSMETIC AGENT CONSISTING OF GALACTOMANNANS AND CROSS-LINKED SULPHATED GALACTANS

(30) Priorité: 29.01.2016 FR 1650736
(43) Date de publication de la demande: 05.12.2018
(73) Titulaire: Societe Industrielle Limousine d'Application Biologique, 19130 Objat (FR)
(72) Inventeur: PAUFIQUE, Jean, 19130 Objat (FR)
(74) Mandataire: Aquinov
(86) Numéro de dépôt international: PCT/EP2017/051849
(87) Numéro de publication internationale: WO 2017/129792

(56) Documents cités:
- EP-A1- 1 559 417
- EP-A2- 0 730 867
- FR-A1- 2 881 349
- FR-A1- 2 986 430
- FR-A1- 3 018 448
- US-A1- 2002 076 769
- US-A1- 2005 118 130
- M D Guiry ET AL: "Kappaphycus alvarezii (Doty) Doty ex P.C.Silva :: Algaebase", AlgaeBase, 26 février 2013 (2013-02-26), pages 1-4, XP55282920, ireland Extrait de l'Internet: URL:http://www.algaebase.org/search/specie s/detail/?species_id=W5ac9a714e03445eb&sk= 0&from=results [extrait le 2016-06-22]
- LABORATORIUM KOSMETYCZNE FLOSLEK: "Dermal Filler Day Cream SPF 15", GNPD, 5 février 2014 (2014-02-05), XP002759145, [extrait le 2014-06-01]
- Anonymous: "Hydromanil", , 1 janvier 2005 (2005-01-01), pages 1-17, XP055115308, Extrait de l'Internet: URL:http://www.innovadex.com/documents/524 153.pdf?bs=2443&b=71056&st=1&sl=28547834&c rit=a2V5d29yZDpbaHlkcm9tYW5pbF0=&k=hydroma nil [extrait le 2014-04-25]
- Nn: "Telosomyl (TM)", , 1 avril 2012 (2012-04-01), pages 1-2, XP055350323, Extrait de l'Internet: URL:https://cdn.shopify.com/s/files/1/0319 /8073/files/Telosomyl-presentation.pdf [extrait le 2017-02-28]
- PARKER A ET AL: "Binding of galactomannans to kappa-carrageenan after cold mixing", CARBOHYDRATE RESEARCH, PERGAMON, GB, vol. 272, no. 1, 21 juillet 1995 (1995-07-21), pages 91-96, XP004021995, ISSN: 0008-6215, DOI: 10.1016/0008-6215(95)00042-R
- FERNANDES P B ET AL: "A rheological characterization of kappa-carrageenan/galactomannan mixed gels: A comparison of locust bean gum samples", CARBOHYDRATE POLYMERS, APPLIED SCIENCE PUBLISHERS, LTD. BARKING, GB, vol. 16, no. 3, 1 janvier 1991 (1991-01-01), pages 253-274, XP024147756, ISSN: 0144-8617, DOI: 10.1016/0144-8617(91)90112-P [extrait le 1991-01-01]

## Description

La présente invention concerne des agents cosmétiques particuliers tels que définis dans la revendication 1 présentant un effet tenseur et/ou filmogène et leur utilisation pour des applications cosmétiques ou dermocosmétiques.

Les femmes sont en quête perpétuelle de solutions visant à garder leur peau jeune et en bonne santé. Elles recherchent le produit capable d'effacer les signes du temps et de leur permettre de se protéger des effets néfastes d'un environnement toujours plus agressif. En effet, en raison de l'industrialisation et du grossissement des villes, les individus sont régulièrement exposés à des allergènes, des molécules irritantes ou des particules fines. Ces dernières notamment émises par les véhicules ont des effets dévastateurs sur la santé. Au-delà de leur incidence au niveau respiratoire, elles entrainent des dégâts majeurs sur la peau en accélérant son vieillissement : apparition de rides et de taches pigmentaires, peau relâchée, perte d'élasticité et manque d'éclat.

Face à cette problématique, le marché des cosmétiques propose une large gamme de soins anti-âge et protecteur. On trouve des produits visant à remodeler en profondeur et sur le long terme la peau afin de lui redonner sa jeunesse. De plus, un éventail d'antioxydants, de détoxifiants ou de stimulateurs des défenses naturelles est à disposition pour aider la peau à se protéger.

Toutefois, en complément de ces traitements « longue durée », les consommateurs souhaitent aussi des résultats immédiats. Les soins intègrent donc dorénavant et très largement des actifs liftants capables d'améliorer instantanément les signes du vieillissement. Depuis 2010, on voit progresser dans les soins de peau, une nouvelle catégorie d'actifs : les actifs « seconde peau ». Ces derniers dotés d'une action à court terme sont intégrés pour leur action coup d'éclat et protecteur face aux agressions extérieures.

Le produit "Dermal Filler Day Cream SPF 15" (Mintel GNPD Record ID 2482521, 06/2014) est une crème anti-rides comprenant une combinaison d'actifs, à savoir "hydrolyzed Caesalpinia spinosa gum" et par conséquent des galactomannanes à masse molaire réduite, ainsi que "Chondrus crispus (extract)", qui contient forcément du carraghène (i.e. des galactanes sulfatés).

L'objectif de la présente invention est de proposer de nouveaux agents cosmétiques avec un effet tenseur perceptible et un effet film « seconde peau » protecteur et liftant capable de booster la jeunesse cutanée.

A cet effet, l'invention vise des agents cosmétiques ou dermocosmétiques constitués par l'association de biopolymères spécifiques, des galactomannanes de masse molaire sélectionnée et des galactanes sulfatés réticulés de masse molaire sélectionnée.

Ainsi, l'invention a pour objet un agent cosmétique ou dermocosmétique constitué par :
- des galactomannanes de masse molaire moyenne comprise entre 5 et 120kDa, et
- des galactanes sulfatés réticulés de masse molaire moyenne comprise entre 7 1100kDa,
lesdits galactomannanes sont obtenus par hydrolyse de galactomannanes natifs de *Caesalpinia spinosa,* de *Cyamopsis tetragonoloba,* et/ou de *Ceratonia siliqua* et lesdits galactanes sulfatés réticulés sont obtenus par hydrolyse de galactanes sulfatés natifs de *Kappaphycus alvarezii, d'Eucheuma cottonii,* et/ou de *Chondrus crispus.*

Plus préférentiellement l'invention a pour objet un agent cosmétique ou dermocosmétique constitué par :
- des galactomannanes de masse molaire moyenne comprise entre 8 et 80kDa, et
- des galactanes sulfatés réticulés de masse molaire moyenne comprise entre 8 et 200kDa.

Ces biopolymères présentent des propriétés biomécaniques et filmogènes puissantes qui confèrent une efficacité de seconde peau protectrice. Avantageusement, ils sont réalisés sans utilisation d'agents chimiques et répondent aux exigences d'écoconception. Avec ces biopolymères particuliers, la peau est protégée, elle retrouve éclat et attractivité. Les signes du vieillissement sont instantanément gommés.

L'invention a donc également pour objet l'utilisation cosmétique de cet agent cosmétique. L'invention vise aussi les compositions cosmétiques incluant l'agent cosmétique selon l'invention ainsi qu'un procédé de traitement cosmétique de la peau utilisant ces compositions.

D'autres caractéristiques et avantages ressortiront de la description en détail de l'invention qui va suivre en regard des figures annexées sur lesquelles :
- la Figure 1 représente un chromatogramme d'un exemple d'agent selon l'invention ;
- les Figures 2A à 2E représentent l'échelle de « - » (Figure 2E) à « ++++ » (Figure 2A) permettant d'évaluer la force de rétractation d'un agent selon l'invention.

### DEFINITIONS

Par « agent cosmétique ou dermocosmétique » ou « agent » au sens de l'invention, on entend un ingrédient, un principe actif ou un excipient adapté à une utilisation dans une composition cosmétique ou dermocosmétique. L'agent selon l'invention est constitué de biopolymères, et peut-être également désigné dans la présente demande par le terme « biopolymère ».

Par « biopolymères », au sens de l'invention, on entend des polymères issus de matières premières végétales, par opposition aux polymères synthétiques, qui sont obtenus par synthèse chimique.

Par « effet filmogène » on entend un effet pouvant créer à la surface de la peau un film non perceptible à l'œil nu, et ainsi protéger la peau des agressions externes telle que la pollution et les allergènes.

Par « effet tenseur », on entend un effet de tension sur la peau et par cet effet de tension, lisser la peau et faire diminuer de façon immédiate les rides et ridules.

Par « filmogène », au sens de l'invention, on entend un biopolymère présentant un effet filmogène, c'est-à-dire tout biopolymère soluble dans l'eau à la concentration de 7% en poids ou à la concentration maximale à laquelle il forme un milieu d'apparence homogène et produisant une fois séché pendant 24h à 40°C, un film qui nécessite une masse d'au moins 100g pour le rompre dans le test décrit dans la présente demande.

Par « tenseur », au sens de l'invention, on entend un biopolymère présentant un effet tenseur, c'est-à-dire tout biopolymère soluble dans l'eau à la concentration de 7% en poids ou à la concentration maximale à laquelle il forme un milieu d'apparence homogène et produisant à cette concentration, une rétractation notée au moins « +++ » dans le test décrit dans la présente demande.

Par « masse molaire moyenne » d'un mélange de molécules au sens de l'invention on entend la moyenne des masses molaires pondérales de chaque molécule du mélange.

On entend par « milieu d'apparence homogène » un milieu ne présentant pas d'agrégats visibles à l'œil nu.

Par « réticulé » au sens de l'invention, on entend un biopolymère dans lequel a été formé un réseau tridimensionnel au moyen de la formation de liaisons chimiques ou physiques entre les molécules du biopolymère.

### DESCRIPTION DETAILLEE DE L'INVENTION

L'invention vise donc un agent cosmétique ou dermocosmétique constitué par :
- des galactomannanes de masse molaire moyenne comprise entre 5 et 120kDa, et
- des galactanes sulfatés réticulés de masse molaire moyenne comprise entre 7 et 1100kDa,
lesdits galactomannanes sont obtenus par hydrolyse de galactomannanes natifs de *Caesalpinia spinosa,* de *Cyamopsis tetragonoloba,* et/ou de *Ceratonia siliqua* et lesdits galactanes sulfatés réticulés sont obtenus par hydrolyse de galactanes sulfatés natifs de *Kappaphycus alvarezii, d'Eucheuma cottonii,* et/ou de *Chondrus crispus.*

Il s'agit donc de biopolymères particuliers sélectionnés avec des tailles spécifiques sélectionnées.

Préférentiellement :
- les galactomannanes présentent une masse molaire moyenne comprise entre 8 et 80kDa,
- les galactanes sulfatés réticulés présentent une masse molaire moyenne comprise entre 8 et 200kDa.

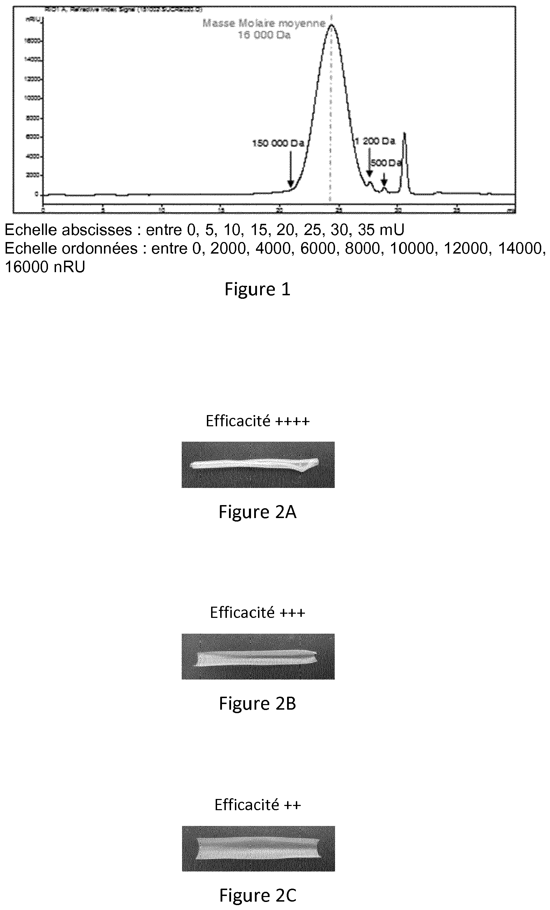

La détermination des masses molaires de ces biopolymères de nature saccharidique, est préférentiellement réalisée par chromatographie d'exclusion stérique. Cette méthode de chromatographie liquide permet de séparer les macromolécules en fonction de leur volume hydrodynamique (chromatographie d'exclusion stérique). Les solutés sont élués dans l'ordre des masses molaires décroissantes après passage sur 3 colonnes de perméation sur gel montées en série (colonnes PL aquagel-OH C60, C40 et C30). Les composés sont détectés par un détecteur à indice de réfaction. Les masses molaires des carbohydrates sont évaluées par comparaison des temps de rétention des pics détectés dans les agents selon l'invention avec les temps de rétention de standards. La masse molaire moyenne d'un agent correspond à la moyenne des masses molaires pondérée par l'intensité de chacune. Par exemple, l'agent dont le chromatogramme est représenté à la Figure 1 contient des biopolymères de masses molaires entre 1,2 à 150kDa, et de masse molaire moyenne de 16kDa.

D'un point de vue physique, les biopolymères selon l'invention présentent préférentiellement une faible viscosité. La viscosité est mesurée à température ambiante à l'aide d'un viscosimètre Brookfield Modèle DV-I+. Le viscosimètre Brookfield détermine la viscosité d'un fluide à partir de la déformation exercée sur un ressort créé par la rotation d'un disque dans ce fluide. On considère qu'un produit est visqueux, si sa viscosité excède 1000 centipoises.

Par ailleurs, ces biopolymères peuvent être définis comme présentant un effet tenseur. Cet effet tenseur peut être défini par la caractérisation de la force de rétractation sur un modèle de peau synthétique ou par une étude sensorielle sur un panel d'experts sensoriels.

La force de rétractation du polymère peut être caractérisée par un test in vitro. Ce modèle est connu de l'homme de métier et a été décrit dans la demande de brevet EP1944065.

Le test est réalisé sur une solution homogène de biopolymères dilués dans l'eau à une concentration de 7% en poids. Le mélange homogène est déposé en un film sur une peau synthétique d'une épaisseur d'environ 100 µm et de largeur initiale de 10 mm. Après séchage à 22 ± 3°C et 40 ± 10% d'humidité relative, la peau synthétique présente une largeur rétractée due à la tension exercée par le polymère déposé.

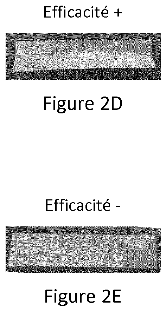

Cette rétractation est évaluée visuellement et quantifiée sur une échelle de « - » à « ++++ », qui est représentée sur les Figures 2A à 2E.

Un produit est considéré comme tenseur avec ce test, si la rétractation est notée au moins « +++ ».

L'effet tenseur peut être évalué également par une étude sensorielle sur un panel d'experts sensoriels. L'étude peut être réalisée en formulant les agents à tester en gel.

Le gel a été réalisé en utilisant la formule suivante :
- agent selon l'invention : 0,50%
- conservateur : 0,23%
- carbomer (Ultrez 10, Noveon) : 0,27%
- Eau : qsp 100%

### (Pourcentages donnés en masse).

Chaque expert note sur une échelle allant de 0 à 10 (0 : pas d'effet tenseur perçu, 10 : effet tenseur important) l'intensité de la sensation perçue après l'application-du produit (agent selon l'invention ou placebo) au niveau de la patte d'oie. La notation est réalisée 3 minutes, 5 minutes et 10 minutes après l'application du produit.

La moyenne des scores obtenus à chaque temps est calculée pour chaque expert.

Un produit est considéré comme tenseur, si la moyenne des scores obtenus est supérieure à 3.

Enfin, l'effet filmogène des agents selon l'invention peut être caractérisé au moyen d'un texturomètre. Le principe est de quantifier l'effet filmogène des échantillons à tester en leur appliquant une pression jusqu'à la rupture du film.

Les échantillons sont préparés comme suit :
- Séchage des solutions contenant les agents selon l'invention à 7% (w/w) pendant 24h à 40°C,
- Obtention de films d'une épaisseur entre 30 et 40µm.

Les échantillons à tester sont déposés à la surface d'une mousse souple et déformable, qui permet d'imposer une déformation importante au film de biopolymères. Le substrat est constitué d'une mousse uréthane élastomère de 5 mm d'épaisseur, il mime la surface et l'élasticité de la peau.

L'étude peut être réalisée avec un Texturomètre TA-XTplus de la société Stable Micro System. Un poinçon cylindrique exerce une contrainte mécanique sur l'échantillon selon une vitesse de déplacement constante.

Une courbe du poids exercé (g) en fonction du temps (sec) est obtenue, à partir de laquelle il est possible de déterminer la masse nécessaire pour obtenir la rupture du film.

La masse nécessaire pour rompre les échantillons, dépend des propriétés viscoélastiques de chaque échantillon. On considère qu'un produit est filmogène si la masse exercée pour rompre le film du produit est supérieure à 100g.

L'agent cosmétique ou dermocosmétique selon l'invention contient donc des galactomannanes de masse molaire moyenne comprise entre 5 et 120kDa, préférentiellement entre 8 et 80kDa.

Les galactomannanes sont connus comme étant des émulsifiants, des épaississants largement utilisés en cosmétique et en alimentaire. Ce sont des polysaccharides d'une taille très élevée (environ 3000kDa).

La viscosité de ces polysaccharides est proportionnelle à la taille des polysaccharides, et elle apporte l'effet d'épaississement.

Les biopolymères de l'invention sont composés des galactomannanes, préférentiellement sélectionnés par leur masse molaire moyenne comprise entre 5 et 120kDa, c'est-à-dire qu'ils sont obtenus préférentiellement par transformation de galactomannanes natifs (d'une taille très élevée) en particulier par hydrolyse.

Ils peuvent être obtenus notamment à partir de galactomannanes de Tara (*Caesalpinia spinosa*)*,* de guar (*Cyamopsis tetragonoloba*)*,* de caroube (*Ceratonia siliqua*)*,* mais aussi de séné (*Cassia angustifolia*)*,* de cassier (*Cassia fistula*)*,* de cassia (*Cassia obtusifolia ou Cassia foro*)*,* de caroube chinois (*Gleditsia sinensis*)*,* de févier (*Gleditsia triacanthos*)*,* de sophora (*Sophora japonica*) et/ou de fenugrec (*Trigonella foenum-graecum*)*.* Dans le contexte de l'invention, ils sont obtenus par hydrolyse de galactomannanes natifs de *Caesalpinia spinosa,* de *Cyamopsis tetragonoloba,* et/ou de *Ceratonia siliqua.*

Les galactomannanes particuliers, sélectionnés selon l'invention sont réalisés comme suit :
- Solubilisation de poudre de galactomannanes natifs dans l'eau à raison d'au moins 20g/l,
- Hydrolyse ménagée par voie chimique ou enzymatique ; la taille des biopolymères selon l'invention est inversement proportionnelle à la durée de l'hydrolyse, ou à la concentration d'enzyme ou d'agent chimique utilisé.
- Séparation des phases soluble et insoluble, afin d'éliminer la phase insoluble,
- Sélection par filtration(s) membranaire(s) des galactomannanes de masse molaire moyenne comprise entre 5kDa et 120kDa.

On obtient un produit A liquide peu visqueux contenant des galactomannanes sélectionnés de masse molaire moyenne comprise entre 5kDa et 120kDa. Ces galactomannanes sélectionnés présentent préférentiellement une masse molaire moyenne comprise entre 8 et 80kDa.

La taille des galactomannanes sélectionnés est déterminée par chromatographie d'exclusion stérique.

L'effet tenseur est évalué sur un modèle de rétractation sur peau synthétique ou par un panel d'experts sensoriels.

La viscosité est déterminée par un viscosimètre.

La force provoquant la rupture du film réalisé avec les principes actifs est évaluée par un texturomètre et est exprimée en masse.

Les résultats caractéristiques de plusieurs exemples d'agents selon l'invention constitués de galactomannanes de masse molaire moyenne comprise entre 5 et 120kDa d'une part et de galactomannanes de masse molaire moyenne en dehors de celle objet de l'invention sont présentés dans le Tableau 1 ci-dessous :

**Tableau 1**

| Masses molaires mini et maxi | Masse molaire moyenne | Viscosité (CP) ou état physique | Effet tenseur sur modèle de rétractation | Effet tenseur sensoriel | Masse entraînant la rupture du film (g) |
|---|---|---|---|---|---|
| | 3 kDa de CeS | 3.4 | - | NT** | NT** |
| | 4.7kDa de CaS | Liquide | - | | |
| 0.5 à 40kDa | 5,4kDa de CaS | 70 | + | NT** | NT** |
| | 7.2kDa de CaS | liquide | + | | |
| 0.7 à 62kDa | 9.5kDa de CaS | liquide | ++ | | |
| | 10kDa de CaS | 85 | +++ | NT** | NT** |
| 0.9 à 117kDa | 13.5kDa de CaS | liquide | +++ | | |
| | 18kDa de CaS | 90 | +++ | 4.5 | 280 |
| | 18.9kDa de CeS | 184 | +++ | | |
| 1.2 à 171kDa | 19kDa de CaS | liquide | +++ | | |
| 1.5 à 215kDa | 22kDa de CaS | liquide | +++ | | |
| 1.6 à 160kDa | 25kDa de CaS | liquide | +++ | | |
| | 63kDa de CyT | 140 | +++ | 4.4 | 410 |
| | 80kDa de CyT | | +++ | | |
| | 117kDa de CyT | NM* | ++ | NT** | NT** |
| | 125-130kDa de CyT | | + | | |
| | 150kDa de CyT | NM* | + | NT** | NT** |
| | 180kDa de CyT | NM* | - | NT** | NT** |
| Gomme CaS non hydrolysée | 1980kDa de CaS | >3125 | - | NT** | NT** |

| | | | | | |
|---|---|---|---|---|---|
| NM* : non mesuré; NT** : non testé *CaS : Caesalpinia spinosa* *CeS : Ceratonia siliqua* *CyT : Cyamopsis tetragonoloba* | | | | | |

Ces résultats montrent bien que les galactomannanes de haute masse molaire moyenne (> 120kDa) et les galactomannanes de faible masse molaire moyenne (<5kDa) ne présentent pas l'effet tenseur recherché sur le modèle de rétractation.

L'effet tenseur visualisé sur le modèle de rétractation sur peau synthétique est corrélé par l'évaluation de l'effet tenseur perçu par un panel d'experts sensoriels. Ceci confirme que les galactomannanes sélectionnés selon l'invention, peuvent être détectés comme tenseur sur le modèle de rétractation, ou par un panel d'experts sensoriels.

Enfin, les galactomannanes de masse molaire sélectionnée présentent bien un effet filmogène mesuré au texturomètre.

L'agent cosmétique ou dermocosmétique selon l'invention contient aussi des galactanes sulfatés réticulés de masse molaire moyenne comprise entre 7 et 1100kDa.

Les galactanes sulfatés sont connus comme étant des émulsifiants, des épaississants largement utilisés en cosmétique et en alimentaire. Ce sont des polysaccharides d'une taille très élevée (jusqu'à 20 000kDa).

La viscosité de ces polysaccharides est proportionnelle à la taille des polysaccharides, et elle apporte l'effet d'épaississement.

Les biopolymères de l'invention sont donc composés de galactanes sulfatés réticulés, préférentiellement sélectionnés par leur masse molaire moyenne comprise entre 7 et 1100kDa, c'est-à-dire qu'ils sont obtenus préférentiellement par transformation de galactanes sulfatés natifs (de taille très élevée), en particulier par hydrolyse.

Ils peuvent être obtenus notamment à partir de galactanes sulfatés de carraghénanes (de *Kappaphycus alvarezii,* de *Kappaphycus striatum, d'Eucheuma cottonii, d'Eucheuma spinosum,* de *Chondrus crispus,* de *Gigartina skottsbergii,* de *Sarcothalia crispata*)*,* ou de *Fucellaria fastigiata,* d'Agar (*Gelidium sesquipedale*) ou des algues (*Polysiphonia lanosa* ou *Codium fragile*)*.* Dans le contexte de l'invention, ils sont obtenus par hydrolyse de galactanes sulfatés natifs de *Kappaphycus alvarezii, d'Eucheuma cottonii,* et/ou de *Chondrus crispus.*

Les galactanes sulfatés particuliers, sélectionnés selon l'invention sont réalisés comme suit :
- Solubilisation de poudre de galactanes sulfatés natifs dans l'eau à raison d'au moins 20g/l,
- Hydrolyse ménagée enzymatique ou chimique ; la taille des biopolymères selon l'invention est inversement proportionnelle à la durée de l'hydrolyse ou à la concentration d'enzyme ou d'agent chimique utilisé,
- Séparation des phases solubles et insolubles, afin d'éliminer la phase insoluble,
- Sélection des galactanes sulfatés réticulés de masse molaire moyenne comprise entre 7kDa et 1100kDa par filtration(s) membranaire(s).

Ces galactanes sulfatés réticulés sélectionnés par leur masse molaire moyenne sont aussi réticulés par un agent réticulant, préférentiellement un agent réticulant de nature ionique. L'agent de réticulation ionique est choisi parmi des cations mono ou multivalents. D'autres agents de réticulation ionique connus de l'homme de métier peuvent être envisagés.

On obtient un produit B liquide ou peu visqueux contenant des galactanes sulfatés réticulés sélectionnés de masse molaire moyenne comprise entre 7kDa et 1100kDa. Ces galactanes sulfatés réticulés sélectionnés présentent préférentiellement une masse molaire moyenne comprise entre 8 et 200kDa.

La caractérisation de la taille des galactanes sulfatés réticulés est réalisée par chromatographie d'exclusion stérique.

L'effet tenseur est évalué sur le modèle de rétractation sur peau synthétique ou par un panel d'experts sensoriels.

La viscosité est déterminée par un viscosimètre.

Les résultats caractéristiques de plusieurs exemples d'agents selon l'invention constitués de galactanes sulfatés de masse molaire moyenne comprise entre 8 et 200kDa d'une part et de galactanes sulfatés de masse molaire moyenne en dehors de celle objet de l'invention sont présentés dans les Tableaux 2a (non réticulés) et 2b (réticulés) ci-dessous :

**Tableau 2a**

| Masses molaires mini maxi | Masse molaire moyenne | Viscosité (CP) ou état physique | Effet tenseur sur modèle de rétractation | Effet tenseur sensoriel |
|---|---|---|---|---|
| 0.18 à 8.1kDa produit FR2986430 | Entre 1.08 et 3.24kDa | liquide | - | |
| | 3,6kDa de KA | 2,2 | - | NT** |
| 0.9 à 36.3kDa | 5,4kDa de KA | 15 | + | NT** |
| 1.3 à 66.1kDa | 10,8kDa de KA | 300 | +++ | 3,6 |
| 1.8 à 150kDa | 14.4kDa de KA | Liquide | +++ | |
| | 15kDa de CC | Liquide | +++ | |
| 2.2 à 97.5kDa | 18kDa de KA | liquide | +++ | NT** |
| | 40kDa de CC | Liquide | +++ | |
| | 79kDa de CC | Liquide | +++ | NT** |
| | 185kDa de CC | 87 | +++ | NT** |
| | 400kDa de CC | Peu visqueux | +++ | |
| | 600kDa de CC | Peu visqueux | ++ | |
| | 1100kDa de CC | Visqueux | ++ | NT** |
| Kappa carraghénanes non hydrolysés | 1500kDa de KA | Très visqueux | - | NT** |
| | 1500kDa de CC | Très visqueux | - | NT** |
| Chondrus crispus non hydrolysés | 3000kDa de CC | Très visqueux | - | NT** |

| | | | | |
|---|---|---|---|---|
| NM* : non mesuré; NT** : non testé KA = *Kappaphycus alvarezii* CC = *Chondrus crispus* | | | | |

Ces résultats montrent bien que les galactanes sulfatés natifs de masse molaire moyenne importante (> 1100kDa) et les galactanes sulfatés de faible masse molaire moyenne (<7kDa) ne présentent pas l'effet tenseur recherché.

Préférentiellement, les galactanes sulfatés réticulés avec une masse molaire moyenne comprise entre 8 et 200kDa présentent bien un effet tenseur visualisé par une force de rétractation et l'état physique recherché.

Les galactanes sulfatés sélectionnés selon l'invention peuvent être détectés comme tenseur sur le modèle de rétractation, ou par un panel d'experts sensoriels.

**Tableau 2b**

| Masse molaire moyenne | Réticulation | Viscosité (CP) ou aspect physique | Effet tenseur sur modèle de rétractation | Effet tenseur sensoriel |
|---|---|---|---|---|
| < 3.24kDa | Non (Produit FR2986430) | Liquide | - | |
| | Oui - agent 1 | Liquide | - | |
| | Oui - agent 2 | Liquide | - | |
| | Oui - agent 3 | Liquide | - | |
| 3,6kDa de KA | Non | 2,2 | - | NT** |
| | Oui - agent 1 | liquide | - | NT** |
| | Oui - agent 2 | liquide | - | NT** |
| | Oui - agent 3 | liquide | - | NT** |
| 10,8kDa de KA | Non | 300 | +++ | 3,6 |
| | Oui - agent 1 | liquide | +++ | NT** |
| | Oui - agent 2 | liquide | +++ | NT** |
| | Oui - agent 3 | liquide | ++++ | 4,5 |
| | Oui - agent 4 | | ++++ | |
| 18kDa de KA | Non | liquide | +++ | NT** |
| | Oui - agent 3 | liquide | +++ | NT** |
| 79kDa de CC | Non | liquide | +++ | NT** |
| | Oui - agent 1 | liquide | ++++ | NT** |
| | Oui - agent 2 | liquide | +++ | NT** |
| | Oui - agent 3 | liquide | +++ | NT** |
| 3000kDa de CC | Non | 100 | - | NT** |
| | Oui - agent 1 | NM* | - | NT** |
| | Oui - agent 2 | NM* | - | NT** |
| | Oui - agent 3 | NM* | - | NT** |

| | | | | |
|---|---|---|---|---|
| NM* : non mesuré ; NT** : non testé Agent 1 et 2 sont des agents de réticulation ionique type cations bivalents Agent 3 est un agent de réticulation ionique type cation monovalent Agent 4 est un agent de réticulation ionique type cation multivalent | | | | |

Ces résultats montrent bien que les galactanes sulfatés natifs de masse molaire moyenne importante (> 1100kDa) et les galactanes sulfatés de faible masse molaire moyenne (<7kDa) ne présentent pas l'effet tenseur recherché. La réticulation ionique ne leur confère pas d'effet tenseur.

Les galactanes sulfatés réticulés, avec une masse molaire moyenne comprise entre 7 et 1100kDa présentent bien un effet tenseur visualisé par une force de rétractation.

Les galactanes sulfatés réticulés sélectionnés selon l'invention, peuvent être détectés comme tenseur sur le modèle de rétractation, ou par un panel d'experts sensoriels.

La réticulation ionique des fonctions sulfates potentialise l'effet tenseur des galactanes sulfatés sélectionnés, mais ne confère pas un effet tenseur à des galactanes sulfatés de masse molaire moyenne trop faible ou trop importante ne possédant pas d'effet tenseur.

L'agent cosmétique ou dermocosmétique selon l'invention est constitué par l'association des galactomannanes de masse molaire moyenne comprise entre 5 et 120kDa, tels que décrits précédemment, et par des galactanes sulfatés réticulés de masse molaire moyenne comprise entre 7 et 1100kDa tels que décrits précédemment ; les galactomannanes sont obtenus par hydrolyse de galactomannanes natifs de *Caesalpinia spinosa,* de *Cyamopsis tetragonoloba,* et/ou de *Ceratonia siliqua* et les galactanes sulfatés réticulés sont obtenus par hydrolyse de galactanes sulfatés natifs de *Kappaphycus alvarezii, d'Eucheuma cottonii,* et/ou de *Chondrus crispus.*

Le procédé de réalisation d'un tel agent comprend les étapes suivantes :
- obtention des galactomannanes de masse molaire moyenne comprise entre 5 et 120kDa, noté produit A, selon le protocole décrit précédemment,
- obtention des galactanes sulfatés réticulés de masse molaire moyenne comprise entre 7 et 1100kDa, noté produit B, selon le protocole décrit précédemment,
- mélange du produit A et du produit B. En particulier, l'agent cosmétique ou dermocosmétique selon l'invention peut être constitué de :
   ∘ 60 à 90% de galactomannanes (produit A), et
   ∘ 10 à 40% de galactanes sulfatés (produit B).

Préférentiellement les galactomannanes et les galactanes sulfatés réticulés forment ensemble un réseau interpénétré.

Plusieurs agents selon l'invention ou hors de l'invention, sous forme de solutions, ont été testés à 7% sur le modèle de peau synthétique et à 0.5% dans l'étude tenseur sensorielle. Les résultats sont présentés dans les Tableaux ci-dessous :
- Les combinaisons avec 80% de galactomannanes et 20% de galactanes sulfatés réticulés dans le Tableau 3a,
- Les combinaisons avec des galactomannanes et des galactanes sulfatés réticulés de masses molaires sélectionnées selon l'invention dans le tableau 3b.

**Tableau 3a**

| Masses molaires mini-maxi | 20% de Galactanes Sulfatés réticulés | 80% de Galactomannanes | Etat physique | Effet tenseur sur modèle de rétractation | Effet tenseur sur panel sensoriel | Effet filmogène au texturomètre (g) |
|---|---|---|---|---|---|---|
| | < 3.24kDa de KA | 4.7kDa de CaS | liquide | - | | |
| | < 3.24kDa de KA | 19kDa de CaS | liquide | ++ | | |
| 0.7 à 70kDa | 7.2kDa de KA | 10kDa de CaS | liquide | ++ | | |
| | 7.9kDa de KA | 11.7kDa de CaS | liquide | ++++ | | |
| 0.9 à 117kDa | 9.4kDa de KA | 13.3kDa de CaS | liquide | ++++ | | |
| | 12.8kDa de KA | 20.7kDa de CaS | liquide | ++++ | | |
| 1.2 à 171kDa | 14.4kDa de KA | 19kDa de CaS | liquide | ++++ | 5.1 | 371 |
| 0.9 à 117 kDa | 14.8kDa de KA | 13.5kDa de CaS | liquide | ++++ | | |
| 1.5 à 190kDa | 16.4kDa de KA | 20.2kDa de CaS | liquide | ++++ | | |
| | 14.4kDa de KA | 63kDa de CyT | Liquide | ++++ | 5.0 | 466 |
| | 20kDa de CC | 150kDa de CyT | visqueux | +++ | | |
| | 70kDa de CC | 3kDa de CeS | Liquide | + | | |
| | 70kDa de CC | 20kDa de CyT | Peu visqueux | +++ | | |
| | 70kDa de CC | 80kDa de CyT | Peu visqueux | ++++ | | |
| | 70kDa de CC | 150kDa de CyT | Visqueux | +++ | | |
| | 79kDa de CC | 18.9kDa de CeS | Liquide | +++ | | |
| | 220kDa de CC | 3kDa de CeS | visqueux | + | | |
| | 220kDa de CC | 150kDa de CyT | Très visqueux | + | | |

| | | | | | | |
|---|---|---|---|---|---|---|
| NT** : non testé KA : Kappaphycus alvarezii CaS : Caesalpinia spinoa CC : Chondrus cripus CyT CeS | | | | | | |

Ces résultats montrent que la masse molaire moyenne des galactanes sulfatés réticulés (entre 7 et 1100kDa, préférentiellement entre 8 et 200kDa) et la masse molaire moyenne des galactomannanes (entre 5 et 120kDa, préférentiellement entre 8 et 80kDa) est nécessaire pour obtenir l'effet et l'aspect physique (liquide ou peu visqueux) attendus.

La combinaison galactomannanes / galactanes sulfatés réticulés dans lesquels soit les galactanes sulfatés réticulés, soit les galactomannanes, soit les deux présentent des masses molaires moyennes en dehors de la sélection de l'invention, ne permettent pas d'obtenir l'état physique, l'effet tenseur et filmogène attendus.

On constate que l'association de galactanes sulfatés sélectionnés réticulés et de galactomannanes sélectionnés selon l'invention permet non seulement de conserver l'effet tenseur de rétractation des galactanes sulfatés sélectionnés réticulés ou des galactomannanes sélectionnés, mais surtout renforcer l'efficacité tenseur perçue par le panel d'experts sensoriels.

**Tableau 3b**

| Produits B Galactanes Sulfatés réticulés sélectionnés | | Produits A Galactomannanes sélectionnés | | Effet tenseur sur modèle de rétractation | Effet tenseur sur panel sensoriel | Effet filmogène au texturomètre |
|---|---|---|---|---|---|---|
| Teneur | Masse molaire moyenne | Teneur | Masse molaire moyenne | | | |
| 10% | 14.4kDa de KA | 90% | 19kDa de CaS | +++ | 4.4 | |
| 20% | 7.2kDa de KA | 80% | 16kDa de CaS | ++++ | | |
| 20% | 7.9kDa de KA | 80% | 11.7kDa de CaS | ++++ | | |
| 20% | 9.4kDa de KA | 80% | 13.3kDa de CaS | ++++ | | |
| 20% | 12.8kDa de KA | 80% | 20.7kDa de CaS | ++++ | | |
| 20% | 14.4kDa de KA | 80% | 19kDa de CaS | ++++ | 5.1 | 371 |
| 20% | 14.8kDa de KA | 80% | 13.5kDa de CaS | ++++ | | |
| 20% | 14.8kDa de KA | 80% | 18kDa de CeS | +++ | | |
| 20% | 14.4kDa de KA | 80% | 80kDa de CyT | +++ | 5.0 | 466 |
| 20% | 16.4kDa de KA | 80% | 20.2kDa de CaS | ++++ | | |
| 20% | 70kDa de CC | 80% | 18kDa de CeS | +++ | | |
| 30% | 14.4kDa de KA | 70% | 19kDa de CaS | +++ | 3.8 | |
| 35% | 14.4kDa de KA | 65% | 80kDa de CyT | +++ | | |
| 50% | 14.4kDa de KA | 50% | 19kDa de CaS | ++ | | |
| 50% | 14.4kDa de KA | 50% | 80kDa de CyT | ++ | | |
| 50% | 70kDa de CC | 50% | 18kDa de CeS | + | | |

| | | | | | | |
|---|---|---|---|---|---|---|
| NT** : non testé | | | | | | |

Ces résultats montrent l'effet surprenant de l'interaction des deux polysaccharides de tailles sélectionnées. Par exemple pour la combinaison 20% / 80%, l'effet tenseur sur le modèle de rétractation est potentialisé dans la combinaison. Un effet similaire est observé par le panel sensoriel évaluant l'effet tenseur.

Ainsi, les combinaisons contenant entre 10 et 40% de galactanes sulfatés réticulés sélectionnés avec entre 90% et 60% de galactomannanes sélectionnés présentent l'efficacité attendue, un effet tenseur et filmogène.

Par contre, on constate que la combinaison 50%/50% de deux composés de tailles sélectionnées ne présente pas un effet tenseur sur le modèle de rétractation aussi potentialisé que les autres combinaisons selon l'invention.

L'association des galactanes sulfatés réticulés sélectionnés selon l'invention et des galactomannanes sélectionnés selon l'invention permet aussi d'augmenter la résistance du film par rapport à la résistance du film des galactomannanes sélectionnés selon l'invention ou du film des galactanes sulfatés sélectionnés selon l'invention seuls.

L'agent cosmétique ou dermocosmétique selon l'invention peut donc être utilisé pour ses différentes propriétés. En particulier, l'invention vise son utilisation comme agent cosmétique ou dermocosmétique tenseur et/ou filmogène.

Il peut ainsi être utilisé en particulier comme agent :
- pour améliorer l'éclat de la peau et/ou lisser la peau, et/ou
- pour améliorer l'effet barrière de la peau, et/ou
- pour un effet cosmétique film perceptible, afin de sentir l'efficacité tenseur, améliorer l'apparence globale du visage et favoriser la tenue des pigments du maquillage, et/ou
- pour un effet cosmétique ou dermocosmétique protecteur de la peau contre la pénétration de molécules toxiques, telles que les polluants, les allergènes, les métaux lourds ou les irritants.

Du fait de ces différentes efficacités, l'invention vise aussi son utilisation pour lutter contre les manifestations disgracieuses du vieillissement de la peau.

L'invention a donc également pour objet :
- l'utilisation cosmétique d'un agent selon l'invention :
   ∘ comme agent cosmétique tenseur et/ou filmogène,
   ∘ comme agent cosmétique pour améliorer l'éclat de la peau et/ou lisser la peau,
   ∘ comme agent cosmétique pour améliorer l'effet barrière de la peau,
   ∘ comme agent cosmétique pour lutter contre les manifestations disgracieuses du vieillissement de la peau.
- l'agent selon l'invention, pour une utilisation :
   ∘ dans la protection de la peau.
   ∘ dans la protection de la peau contre la pénétration de molécules toxiques.

L'agent cosmétique et/ou dermocosmétique selon l'invention est préférentiellement utilisé dans une composition, cette composition comprenant un milieu cosmétiquement acceptable. Il s'agit de compositions dans différentes formes galéniques, adaptées à l'administration par voie topique sur la peau humaine.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant au moins 0,01% d'un agent selon l'invention, préférentiellement entre 0,05 et 1%.

Ces compositions comprennent, outre l'agent, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les filtres organiques,
- les filtres inorganiques,
- les colorants, les conservateurs, les charges, les pigments, les minéraux,
- les tenseurs,
- les séquestrants,
- les parfums,
- et leurs mélanges, sans que cette liste soit limitative.

Des exemples de tels adjuvants sont cités notamment dans le Dictionnaire CTFA (*International Cosmetic Ingrédient Dictionary and Handbook* publié par le *Personal Care Product Council*)*.*

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour les effets procurés par l'agent cosmétique ou dermocosmétique selon l'invention.

L'invention vise aussi spécifiquement un procédé cosmétique de soin de la peau pour améliorer l'état de la peau, en particulier pour améliorer l'éclat de la peau et/ou lutter contre les manifestations disgracieuses du vieillissement de la peau. Préférentiellement le procédé consiste à appliquer au moins une fois par jour sur la peau du visage une composition comprenant au moins 0,05% en poids de matières sèches de l'agent cosmétique ou dermocosmétique selon l'invention.

Afin d'illustrer l'invention, les exemples suivants avec leurs résultats d'essais sont présentés.

### EXEMPLES

### Exemple 1 : Galactomannanes sélectionnés obtenus à partir de Caesalpinia spinosa

Un exemple d'agent selon l'invention est un agent constitué de galactomannanes de masse molaire moyenne 10kDa, obtenus à partir de Caesalpinia spinosa.

Cet agent peut être obtenu par la mise en oeuvre d'un procédé comprenant les étapes suivantes :
- Solubilisation de poudre de galactomannanes natifs de Caesalpinia spinosa dans l'eau à 20g/l,
- Hydrolyse acide ménagée pendant 2h,
- Décantation afin de séparer les phases soluble et insoluble, et éliminer la phase insoluble,
- Filtration membranaire afin de sélectionner les polysaccharides de masse molaire moyenne de 10kDa.

L'agent obtenu est caractérisé par une masse molaire moyenne de 10kDa.

### Exemple 2 : Galactomannanes sélectionnés obtenus à partir de Ceratonia siliqua

Un exemple d'agent selon l'invention est un agent constitué de galactomannanes de masse molaire moyenne 18,9kDa obtenus à partir de Ceratonia siliqua.

Cet agent peut être obtenu par la mise en oeuvre d'un procédé comprenant les étapes suivantes :
- Solubilisation de poudre de galactomannanes natifs de Ceratonia siliqua natifs dans l'eau à 20g/l,
- Hydrolyse acide ménagée pendant 1h,
- Décantation afin de séparer les phases soluble et insoluble, et éliminer la phase insoluble,
- Filtration membranaire afin de sélectionner les polysaccharides de masse molaire moyenne de 18,9kDa.

L'agent obtenu est caractérisé par une masse molaire moyenne de 18.9kDa.

### Exemple 3 : Galactomannanes sélectionnés obtenus à partir de Cyamopsis tetragonoloba

Un exemple d'agent selon l'invention est un agent constitué de galactomannanes de masse molaire moyenne 63kDa obtenus à partir de Cyamopsis tetragonoloba.

Cet agent peut être obtenu par la mise en oeuvre d'un procédé comprenant les étapes suivantes :
- Solubilisation de poudre de galactomannanes natifs de Cyamopsis tetragonoloba dans l'eau à 20g/l,
- Hydrolyse enzymatique pendant 30mn,
- Décantation pour séparer les phases soluble et insoluble, et éliminer la phase insoluble,
- Filtration membranaire afin de sélectionner les polysaccharides de masse molaire moyenne de 63kDa.

L'agent obtenu est caractérisé par une masse molaire moyenne de 63kDa.

### Exemple 4 : Galactanes sulfatés sélectionnés non réticulés obtenus à partir de kappaphycus alvarezii

Un exemple d'agent selon l'invention est un agent constitué de galactanes sulfatés non réticulés de masse molaire moyenne 10.8kDa, obtenus à partir de kappaphycus alvarezii. Cet agent peut être obtenu par la mise en oeuvre d'un procédé comprenant les étapes suivantes :
- Solubilisation de poudre de galactanes sulfatés natifs de kappaphycus alvarezii dans l'eau à 20g/l,
- Hydrolyse acide ménagée pendant 3h,
- Filtration afin de séparer les phases soluble et insoluble, et éliminer la phase insoluble,
- Filtration membranaire et sélection des polysaccharides de masse molaire moyenne de 10.8kDa.

L'agent obtenu est caractérisé par une masse molaire moyenne de 10.8kDa.

### Exemple 5 : Galactanes sulfatés sélectionnés réticulés obtenus à partir de kappaphycus alvarezi

Un exemple d'agent selon l'invention est un agent constitué de galactanes sulfatés réticulés de masse molaire moyenne 10.8kDa, obtenus à partir de kappaphycus alvarezi.

Cet agent peut être obtenu par la mise en oeuvre d'un procédé comprenant les étapes suivantes :
- Process de l'exemple 4
- Ajout de l'agent de réticulation, CaCl2,
- Filtration afin de séparer les phases soluble et insoluble, et éliminer toute fraction insoluble.

L'agent obtenu est caractérisé par une masse molaire moyenne de 10.8kDa.

### Exemple 6 : Galactanes sulfatés non réticulés obtenus à partir de Chondrus crispus

Un exemple d'agent selon l'invention est un agent constitué de galactanes sulfatés non réticulés de masse molaire moyenne 79kDa, obtenus à partir de *Chondrus crispus.*

Cet agent peut être obtenu par la mise en oeuvre d'un procédé comprenant les étapes suivantes :
- Solubilisation de poudre de galactanes sulfatés natifs de *Chondrus crispus* dans l'eau à 20g/l,
- Hydrolyse enzymatique ménagée pendant 1h,
- Décantation afin de séparer les phases soluble et insoluble, et éliminer la phase insoluble,
- Filtration membranaire pour sélectionner les polysaccharides de masse molaire moyenne de 79kDa.

L'agent obtenu est caractérisé par une masse molaire moyenne de 79kDa.

### Exemple 7 : Galactanes sulfatés sélectionnés réticulés obtenus à partir de Chondrus crispus

Un exemple d'agent selon l'invention est un agent constitué de galactanes sulfatés réticulés de masse molaire moyenne 79kDa, obtenus à partir de *Chondrus crispus.*

Cet agent peut être obtenu par la mise en oeuvre d'un procédé comprenant les étapes suivantes :
- Process de l'exemple 6
- Ajout de l'agent de réticulation, CaCl2,
- Filtration afin de séparer les phases solubles et insolubles, et éliminer toute fraction insoluble.

L'agent obtenu est caractérisé part une masse molaire moyenne de 79kDa.

### Exemple 8 : Combinaison de galactomannanes selon l'invention obtenus à partir de Tara et de galactanes sulfatés réticulés selon l'invention obtenus à partir de kappaphycus alvarezi

Un exemple d'agent selon l'invention est un agent constitué d'un mélange de galactomannanes selon l'invention obtenus à partir de Tara de masse molaire moyenne 10kDa et de galactanes sulfatés réticulés selon l'invention de masse molaire moyenne 10.8kDa obtenus à partir de kappaphycus alvarezi.

Cet agent peut être obtenu par la mise en oeuvre d'un procédé comprenant les étapes suivantes :
- Réalisation des process des exemples 1 et 5,
- Mélange de 90% de l'exemple 1 et 10% de l'exemple 5,

### Exemple 10 : Combinaison selon l'invention de galactomannanes obtenus à partir de Cyamopsis tetragonoloba et de galactanes sulfatés réticulés selon l'invention obtenus à partir de kappaphycus alvarezi

Un exemple d'agent selon l'invention est un agent constitué d'un mélange de galactomannanes selon l'invention obtenus à partir de Cyamopsis tetragonoloba de masse molaire moyenne 63kDa et de galactanes sulfatés réticulés selon l'invention de masse molaire moyenne 10.8kDa obtenus à partir de kappaphycus alvarezi.

Cet agent peut être obtenu par la mise en oeuvre d'un procédé comprenant les étapes suivantes :
- Réalisation des process des exemples 3 et 5,
- Mélange de 65% de l'exemple 3 et 35% de l'exemple 5,
- Filtration membranaire afin d'éliminer les composés insolubles.

### Exemple 10 : Combinaison selon l'invention de galactomannanes obtenus à partir de Caroube et de galactanes sulfatés réticulés selon l'invention obtenus à partir de Chondrus crispus

Un exemple d'agent selon l'invention est un agent constitué d'un mélange de galactomannanes selon l'invention obtenus à partir de Caroube de masse molaire moyenne 18.9kDa et de galactanes sulfatés réticulés selon l'invention de masse molaire moyenne 79kDa obtenus à partir de Chondrus crispus.

Cet agent peut être obtenu par la mise en oeuvre d'un procédé comprenant les étapes suivantes :
- Réalisation des process des exemples 2 et 7,
- Mélange de 80% de l'exemple 2 et 20% de l'exemple 7,
- Filtration membranaire afin d'éliminer les composés insolubles.

Tous ces exemples d'agents présentent des effets filmogènes et tenseurs (voir résultats des Tableaux 1 à 3).

Ces exemples ne sont pas limitatifs et les résultats d'essais avec d'autres exemples (non limitatifs également) ont également été présentés dans les tableaux précédents.

## Revendications

1. Agent cosmétique ou dermocosmétique constitué par :
- des galactomannanes de masse molaire moyenne comprise entre 5 et 120kDa, et
- des galactanes sulfatés réticulés de masse molaire moyenne comprise entre 7 et 1100kDa,
**caractérisé en ce que** les galactomannanes sont obtenus par hydrolyse de galactomannanes natifs de *Caesalpinia spinosa,* de *Cyamopsis tetragonoloba,* et/ou de *Ceratonia siliqua* et **en ce que** les galactanes sulfatés réticulés sont obtenus par hydrolyse de galactanes sulfatés natifs de *Kappaphycus alvarezii, d'Eucheuma cottonii,* et/ou de *Chondrus crispus.*

2. Agent cosmétique ou dermocosmétique selon la revendication précédente, **caractérisé en ce que** les galactomannanes présentent une masse molaire moyenne comprise entre 8 et 80kDa.

3. Agent cosmétique ou dermocosmétique selon l'une des précédentes revendications, **caractérisé en ce que** les galactanes sulfatés réticulés présentent une masse molaire moyenne comprise entre 8 et 200kDa.

4. Agent cosmétique ou dermocosmétique selon l'une des précédentes revendications, **caractérisé en ce que** les galactanes sulfatés sont réticulés avec un agent de réticulation de nature ionique.

5. Agent cosmétique ou dermocosmétique selon l'une des précédentes revendications, **caractérisé en ce qu'**il est constitué de :
- 60 à 90% de galactomannanes, et
- 10 à 40% de galactanes sulfatés réticulés,
les pourcentages étant donnés en masse/masse.

6. Utilisation cosmétique d'un agent selon l'une des précédentes revendications, comme agent cosmétique tenseur et/ou filmogène.

7. Utilisation cosmétique d'un agent selon l'une des revendications 1 à 5, comme agent cosmétique pour améliorer l'éclat de la peau et/ou lisser la peau.

8. Utilisation cosmétique d'un agent selon l'une des revendications 1 à 5, comme agent cosmétique pour améliorer l'effet barrière de la peau.

9. Agent selon l'une des revendications 1 à 5, pour son utilisation dans la protection de la peau contre la pénétration de molécules toxiques.

10. Utilisation d'un agent selon l'une des revendications 1 à 5, comme agent cosmétique pour lutter contre les manifestations disgracieuses du vieillissement de la peau.

11. Composition cosmétique adaptée pour une application topique sur la peau humaine, comprenant au moins 0.01% d'un agent cosmétique ou dermocosmétique selon l'une des revendications 1 à 5.

12. Procédé cosmétique pour améliorer l'état de la peau, **caractérisé en ce qu'**il consiste à appliquer sur la peau une composition cosmétique selon la revendication 11.

13. Procédé cosmétique selon la revendication 12, pour améliorer l'éclat de la peau.

14. Procédé cosmétique selon la revendication 12, pour lutter contre les manifestations disgracieuses du vieillissement de la peau.

## Patentansprüche

1. Kosmetisches oder dermokosmetisches Mittel, bestehend aus:
- Galactomannanen mit mittlerer Molmasse zwischen 5 und 120 kDa, und
- vernetzten sulfatierten Galactanen mit mittlerer Molmasse zwischen 7 und 1100 kDa,
**dadurch gekennzeichnet, dass** die Galactomannane durch Hydrolyse von nativen Galactomannanen von *Caesalpinia spinosa,* von *Cyamopsis tetragonoloba,* und/oder von *Ceratonia siliqua* erhalten werden und dass die vernetzten sulfatierten Galactane durch Hydrolyse von nativen sulfatierten Galactanen von *Kappaphycus alvarezii,* von *Eucheuma cottonii,* und/oder von *Chondrus crispus* erhalten werden.

2. Kosmetisches oder dermokosmetisches Mittel nach dem vorstehenden Anspruch, **dadurch gekennzeichnet, dass** die Galactomannane eine mittlere Molmasse zwischen 8 und 80 kDa aufweisen.

3. Kosmetisches oder dermokosmetisches Mittel nach einem der vorstehenden Ansprüche, **dadurch gekennzeichnet, dass** die vernetzten sulfatierten Galactane eine mittlere Molmasse zwischen 8 und 200 kDa aufweisen.

4. Kosmetisches oder dermokosmetisches Mittel nach einem der vorstehenden Ansprüche, **dadurch gekennzeichnet, dass** die sulfatierten Galactane mit einem Vernetzungsmittel ionischer Natur vernetzt sind.

5. Kosmetisches oder dermokosmetisches Mittel nach einem der vorstehenden Ansprüche,
**dadurch gekennzeichnet, dass** es besteht aus:
- 60 bis 90 % Galactomannanen, und
- 10 bis 40 % vernetzten sulfatierten Galactanen,
wobei die Prozentsätze in Massenprozent angegeben sind.

6. Kosmetische Verwendung eines Mittels nach einem der vorstehenden Ansprüche als straffendes und/oder filmbildendes kosmetisches Mittel.

7. Kosmetische Verwendung eines Mittels nach einem der Ansprüche 1 bis 5 als kosmetisches Mittel zum Verbessern der Ausstrahlung der Haut und/oder zum Glätten der Haut.

8. Kosmetische Verwendung eines Mittels nach einem der Ansprüche 1 bis 5 als kosmetisches Mittel zum Verbessern der Barrierewirkung der Haut.

9. Mittel nach einem der Ansprüche 1 bis 5 zur Verwendung bei dem Schutz der Haut vor dem Eindringen toxischer Moleküle.

10. Verwendung eines Mittels nach einem der Ansprüche 1 bis 5 als kosmetisches Mittel zum Bekämpfen der unschönen Erscheinungen der Hautalterung.

11. Kosmetische Zusammensetzung, die für eine topische Anwendung auf der menschlichen Haut angepasst ist, umfassend mindestens 0,01 % eines kosmetischen oder dermokosmetischen Mittels nach einem der Ansprüche 1 bis 5.

12. Kosmetisches Verfahren zum Verbessern des Hautzustands,
**dadurch gekennzeichnet, dass** es darin besteht, eine kosmetische Zusammensetzung nach Anspruch 11 auf die Haut aufzutragen.

13. Kosmetisches Verfahren nach Anspruch 12 zum Verbessern der Ausstrahlung der Haut.

14. Kosmetisches Verfahren nach Anspruch 12 zum Bekämpfen der unschönen Erscheinungen der Hautalterung.

## Claims

1. A cosmetic or dermocosmetic agent consisting of:
- galactomannans with an average molar mass of between 5 and 120 kDa, and
- crosslinked sulfated galactans with an average molar mass of between 7 and 1100 kDa,
**characterized in that** the galactomannans are obtained by hydrolysis of native galactomannans of *Caesalpinia spinosa, of Cyamopsis tetragonoloba,* and/or of *Ceratonia siliqua* and **in that** the crosslinked sulfated galactans are obtained by hydrolysis of native sulfated galactans of *Kappaphycus alvarezii,* of *Eucheuma cottonii,* and/or of *Chondrus crispus.*

2. The cosmetic or dermocosmetic agent according to the preceding claim, **characterized in that** the galactomannans have an average molar mass of between 8 and 80 kDa.

3. The cosmetic or dermocosmetic agent according to one of the preceding claims, **characterized in that** the crosslinked sulfated galactans have an average molar mass of between 8 and 200 kDa.

4. The cosmetic or dermocosmetic agent according to one of the preceding claims, **characterized in that** the sulfated galactans are crosslinked with an ionic crosslinking agent.

5. The cosmetic or dermocosmetic agent according to one of the preceding claims,
**characterized in that** it consists of:
- 60 to 90% galactomannans, and
- 10 to 40% crosslinked sulfated galactans,
the percentages being given by mass/mass.

6. A cosmetic use of an agent according to one of the preceding claims, as a tightening and/or film-forming cosmetic agent.

7. The cosmetic use of an agent according to one of claims 1 to 5, as a cosmetic agent for improving the radiance of the skin and/or for smoothing the skin.

8. The cosmetic use of an agent according to one of claims 1 to 5, as a cosmetic agent for improving the barrier effect of the skin.

9. The agent according to one of claims 1 to 5, for its use in protecting the skin against the penetration of toxic molecules.

10. The use of an agent according to one of claims 1 to 5, as a cosmetic agent for combating the unsightly manifestations of aging of the skin.

11. A cosmetic composition suitable for topical application to human skin, comprising at least 0.01% of a cosmetic or dermocosmetic agent according to one of claims 1 to 5.

12. A cosmetic method for improving the state of the skin,
**characterized in that** it consists in applying a cosmetic composition according to claim 11 to the skin.

13. The cosmetic method according to claim 12, for improving the radiance of the skin.

14. The cosmetic method according to claim 12, for combating the unsightly manifestations of aging of the skin.
